# EUROPEAN PATENT SPECIFICATION

(11) **EP 4 205 701 B1**
(45) Date of publication and mention of the grant of the patent: **27.05.2026**
(21) Application number: 21879346.1
(22) Date of filing: 11.10.2021
(51) Int. Cl.: A61F 2/07

(54) **COVERED STENT**
ABGEDECKTER STENT
STENT RECOUVERT

(30) Priority: 13.10.2020 CN 202011092050; 13.10.2020 CN 202022275609 U
(43) Date of publication of application: 05.07.2023
(73) Proprietor: Hangzhou Endonom Medtech Co., Ltd, Hangzhou, Zhejiang 310051 (CN)
(72) Inventor: ZHANG, Wayne W., Hangzhou, Zhejiang 310051 (CN)
(74) Representative: MERH-IP Matias Erny Reichl Hoffmann Patentanwälte PartG mbB
(86) International application number: PCT/CN2021/123150
(87) International publication number: WO 2022/078302

(56) References cited:
- WO-A1-2020/125226
- CN-A- 105 832 446
- CN-A- 109 833 116
- CN-A- 109 875 723
- CN-A- 112 022 436
- CN-U- 203 841 852
- CN-U- 210 447 282
- CN-U- 212 438 945
- US-A1- 2011 313 512
- US-A1- 2017 086 993

## Description

### TECHNICAL FIELD

The present invention relates to the technical field of medical devices, particularly to a covered stent.

### DESCRIPTION OF THE PRIOR ART

Aortic aneurysm refers to the abnormal local or diffuse expansion of the aortic wall, which would compress surrounding organs and cause symptoms, with a main risk of aneurysmal rupture. Aortic aneurysm may occur in the ascending aorta, aortic arch, descending thoracic aorta, thoracoabdominal aorta, and abdominal aorta. Aortic aneurysms are classified into true aortic aneurysms, aortic pseudoaneurysms, and dissecting aortic aneurysms according to the structures.

An Aortic aneurysm would cause increased pressure inside the blood vessel, and manifest as progressively swelling. In the case of development for a long time, it will eventually rupture. The larger size of the aneurysm, the higher possibility of rupture. Embolism is another complication. Statistics indicated that without surgical treatment, 90% of patients with thoracic aortic aneurysms would die within 5 years and 75% of patients with abdominal aortic aneurysms would die within 5 years.

The dissecting aortic aneurysm occurs mainly due to focal damage of the arterial intima, and high pressure blood flushes into the vessel wall, causing a tear in the media (generally at an interface of the inner 1/3 and outer 2/3 of the media), which results in that the entire structure of the aortic wall is split, and a dissecting lumen is formed in a splitting space between the inner and outer walls of the dissecting layer. To distinguish the dissecting lumen from the aortic lumen, the dissecting lumen is called a pseudo-lumen, and the aortic lumen is called a true lumen.

Aortic diseases may involve branch arteries, and once the branch arteries are involved, it is difficult to be treated by interventional methods. At present, endovascular aortic repair, i.e., a minimally invasive method, has been developed, in which a stent-graft, namely arterial covered stent, can be inserted into the diseased aorta through the vascular lumen to treat the arterial disease and improve the blood supply, thereby achieving the purpose of treatment. During use, the arterial covered stent is compressed to an axial axis and then loaded into a delivery device, by which the arterial covered stent is delivered, through femoral artery, iliac artery, brachial artery, to a lesion site in the artery and deployed there. Due to the elastic force of the arterial covered stent, it automatically returns to a straight tubular shape and tightly attach to the inner wall of the aorta, to isolate the lesion site in the artery from the blood flow, thus achieving the purpose of treatment.

Due to the limitations of the current stent structure, the existing stent-grafts related to the treatments of arterial branches all have inevitable problems, causing the potential risk of complications during or after the procedures, which are specifically described as follows.

### 1. Chimney stent

The "Chimney" technique is also known as the parallel stent technique, in which a stent is inserted in an occluded branch vessel and released in parallel with the main stent-graft in the aorta to achieve the purpose of preservation the arch branches. The chimney stents include subclavian artery "chimney", carotid artery "chimney" and brachiocephalic trunk artery "chimney" based on different aortic lesions. The chimney stents include "single chimney", "double chimney" and "triple chimney" depending on the number of stents. The advantages of the "chimney stent" technique include that conventional instruments can be used as the surgical instruments which are widely available; the technical difficulty is less challenging; the incidence of endoleak is low and the occlusion effect is good in patients with appropriate selections. However, it has the following disadvantages: a gutter exists between the small stent and main aortic stent-graft, so that the occlusion technique is not perfect, and there is a risk of endoleak. Moreover, since the main aortic stent and the small stent are released in parallel fashion, the main stent-graft may compress the small stent, causing branch stent stenosis and occlusion.

### 2. Fenestrated stent

The existing "fenestration" technique mainly involves in-situ fenestration in surgery and customized fenestration. In terms of in-situ fenestration, the main-body aortic stent-graft is positioned to cover the lesion and the to-be-fenestrated vessel, and then a fenestration instrument (such as a hard-tip guide wire, laser, or radio frequency catheter) is inserted into the to-be-fenestrated branch vessel, to puncture the aortic stent; and then the branch vessel is opened with the aid of a balloon and a stent. The in-situ fenestration procedure involves complicated operations, and has high requirements for the surgeon's skill in surgery. Furthermore, the durability and long-term fatigue resistance of the implanted stent-graft are poor, and endoleak tends to occur. The pre-fenestrated stent-grafts are individually customized by stent manufacturers, and stent-grafts in line with the anatomy of aortic arches of different patients are manufactured based on the preoperative imaging examination results. Therefore, this type of stent-grafts can provide good support, attachment, and positioning ability. However, the customized stent-graft requires a certain period of time to produce, and is not available for emergent situation. In addition, technical details need to be considered in the manufacture of the stent-graft, such as the curvature of the aortic arch, the relative position of the branch openings of the great vessels in the arch, the lesion site, marking of the window, and positioning of the stent-graft.

### 3. Integral branched stent

The integral branched stent-graft can avoid the gutter associated with chimney technique and the problems of endoleak and others caused by poor alignment of the window, and has good attachment performance and thus a low possibility of endoleak. However, affected by numerous technical problems including the delivery, introduction, positioning, and release of the branched stent-graft, up to now, no mature and applicable stent products are available in the market.

A known lumen stent is disclosed in PCT application WO2020125226 A1, which includes a tubular body, and inner branches and outer branches which are respectively in communication with the tubular body. Another known stent graft assembly disclosed in the US Patent Application Publication No. US 20170086993A1 comprises a first tubular body portion, and a second tubular body portion located at least partially distally of first tubular body portion. The stent graft assembly comprises first branch graft, second branch graft, and third branch graft. Therefore, there is a demand on developing an interventional stent-graft system that is more suitable for the reconstruction of branches of the aortic arch.

### SUMMARY OF THE DISCLOSURE

### Technical problem

The objective of the present invention is to develop a covered stent with no need to be customized, which provides a widespread applications and simplify surgical operations. Technical solution

To solve the above technical problems, the following technical solutions are adopted in the present invention. A covered stent includes a stent body, an outer branch, and at least one inner branch. The stent body is a tubular structure covered with membrane on the surface, forming a radially recessed concave portion thereon. The outer branch is overhung outside the stent body, and has one end connected to a side wall of the concave portion, with an inner opening on the side wall of the concave portion. The inner branch is attached to an inner wall of the stent body, and each inner branch has one end connected to the side wall of the concave portion with an outer opening on the side wall of the concave portion, wherein the outer opening is closer to a distal end than the inner opening.

### Beneficial effects

The covered stent of the present invention does not need to be customized, and has widespread applications and provide simple procedure for surgical operations.

### BRIEF DESCRIPTION OF DRAWINGS

FIG. 1 is a schematic structural view of a covered stent according to a first embodiment of the present invention.
FIG. 2 is a schematic view of the covered stent shown in FIG. 1, viewed from another aspect.
FIG. 3 is a partially enlarged view of portion A in the covered stent shown in FIG. 2.
FIG. 4 shows the covered stent shown in FIG. 1 in use.
FIG. 5 is a schematic structural view of a covered stent according to a second embodiment of the present invention.
FIG. 6 is a schematic structural view of a covered stent according to a third embodiment of the present invention.
FIG. 7 is a schematic structural view of a covered stent according to a fourth embodiment of the present invention.
FIG. 8 is a schematic structural view of a covered stent according to a fifth embodiment of the present invention.
FIG. 9 is a schematic structural view of a covered stent according to a sixth embodiment of the present invention.
FIG. 10 is a schematic structural view of a covered stent according to a seventh embodiment of the present invention.

List of reference numerals:
100/100a/100b/100c/100d/100e/100f, covered stent; 200, first branch stent; 300, second branch stent; 400, third branch stent; 600, aorta; 601, ascending aorta; 602, aortic arch; 603, descending aorta; 700, innominate artery; 800, left common carotid artery; 900, left subclavian artery;
1/1a/1c/1d/1e/1f, stent body; 101, support frame; 102/102c, covering membrane; 103, concave portion; 104, transition support frame; 105, last-releasing portion; 106, radiopaque marker; 11, proximal tube body; 12, middle tube body; 13, distal tube body; 14, first transition tube body; 15, second transition tube body;
2/2a/2c/2d/2e/2f, outer branch; 21/21a/21f, inner opening; 22/22e/22f, membrane; 221f, flexible section; 23/23e/23f, support ring; 24, marker;
3/3a/3b/3c/3d/3e/3f, first inner branch; 31/31a/31c/31d, first outer opening; 32/32b, marker;
4/4a/4b/4c/4d/4e/4f, second inner branch; 41/41c/41d, second outer opening; 42/42b, marker.

### DESCRIPTION OF EMBODIMENTS

Exemplary embodiments illustrating the features and advantages of the present invention will be described in detail hereinafter. It should be understood that various modifications of the present invention can be made in various embodiments, without departing from the scope of the present invention. The descriptions and accompanying drawings herein are provided substantially for illustration, but are not restrictive to the present invention.

The present invention provides a covered stent, configured to be used for endovascular therapy of aortic arch pathologies. Using the covered stents, the blood flow channels between the aorta and three main branch arteries in aortic arch will be reconstructed.

For the convenience of description, in the context, the term "proximal end" refers to an end adjacent to the heart in the direction of blood flow, and the term "distal end" refers to an end away from the heart, wherein the direction of blood flow in the artery flows from the proximal end to the distal end.

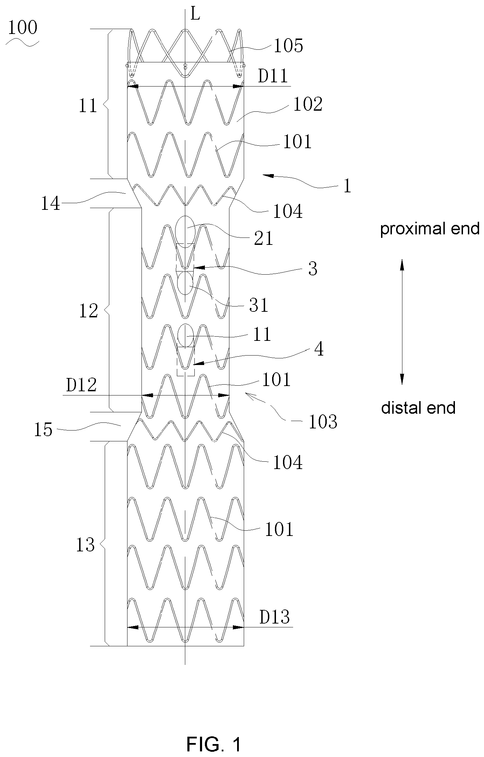

Referring to FIGs. 1 to 4, which show the structure and the use state of a covered stent 100 according to a first embodiment of the present invention.

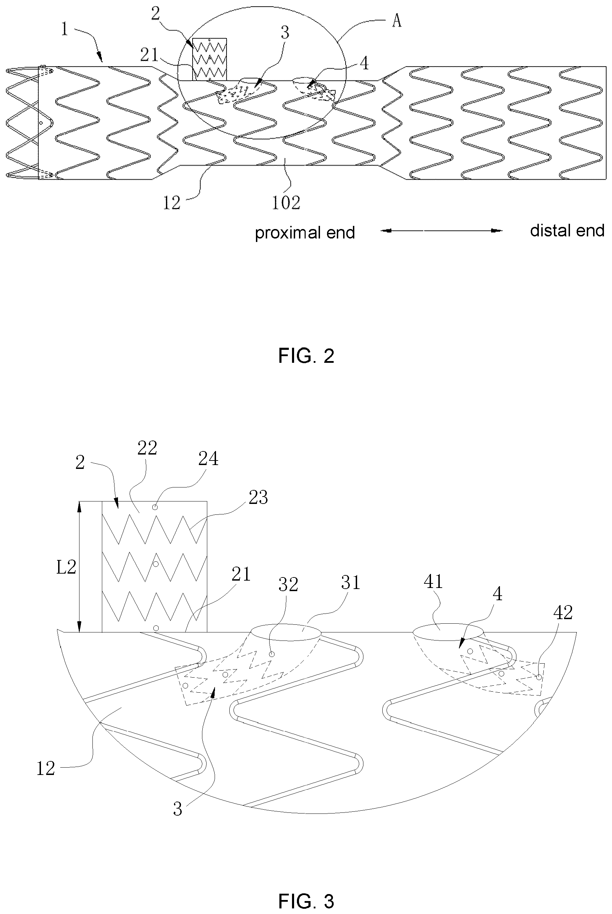

Referring to FIGs. 1 and 2 first, the covered stent 100 according to this embodiment mainly includes a stent body 1, an outer branch 2 and two inner branches 3, 4, wherein the outer branch 2 and two inner branches 3, 4 are integrally connected to the stent body 1. The outer branch 2 is located outside the stent body 1, and the two inner branches 3, 4 are both located inside the stent body 1. The outer branch 2 and the two inner branches 3, 4 are arranged in sequence from the proximal end to the distal end, and fixedly connected to the stent body 1. For the convenience of descriptions, the two inner branches 3, 4 are designated as a first inner branch 3 which is closer to the proximal end and a second inner branch 4 which is closer to the distal end.

Referring mainly to FIG. 1, the stent body 1 is a tubular structure with a covering membrane 102 on the surface, and has a framework substantially composed of a plurality of ring-shaped support frames 101 arranged axially. The support frame 101 is a ring formed of an elastic rigid wire, which is radially collapsible or expandable.

The support frame 101 may be formed of a shape memory alloy material, preferably Nitinol material. The covering membrane 102 can be made from any material suitable for covering membrane, including but not limited to, low-porosity woven or knitted polyester, terylene materials, expanded polytetrafluoroethylene, polyurethane, silicone, ultra-high-molecular-weight polyethylene or other suitable materials.

Each support frame 101 is fixed to an inner or outer surface of the covering membrane 102, such that the covering membrane 102 is supported by the multiple support frames 101. Accordingly, the stent body 1 can expand and maintain as a tubular structure in use, thereby constructing a channel for blood to pass through. Preferably, the support frames 101 are fixed to the covering membrane 102 by suturing or hot-pressing.

In an axial direction of the stent body 1 from the proximal end to the distal end, the stent body 1 has a proximal tube body 11, a middle tube body 12, and a distal tube body 13 in sequence.

In this embodiment, each of the proximal tube body 11, the middle tube body 12 and the distal tube body 13 is a tubular structure with a substantially constant diameter. The diameter D12 of the middle tube body 12 is smaller than the diameter D11 of the proximal tube body 11 and the diameter D13 of the distal tube body 13. Therefore, the entire stent body 1 is radially recessed to form a concave portion 103 at the middle tube body 12.

For example, the diameter D12 of the middle tube body 12 is substantially 70% to 80% of the diameter D11 of the proximal tube body 11, and substantially 75% to 95% of the diameter D13 of the distal tube body 13. It can be understood that the diameter D11 of the proximal tube body 11 may be the same as or different from the diameter D13 of the distal tube body 13.

Preferably, the stent body 1 further includes a first transition tube body 14 located between the proximal tube body 11 and the middle tube body 12.

The first transition tube body 14 is a tapered tubular structure with a diameter varying gradually. A proximal end of the first transition tube body 14 is connected to the proximal tube body 11, and a distal end of the first transition tube body 14 is connected to the middle tube body 12. Accordingly, the proximal diameter of the first transition tube body 14 is greater than the distal diameter.

Preferably, the stent body 1 further includes a second transition tube body 15 located between the middle tube body 12 and the distal tube body 13.

The second transition tube body 15 is also a tapered tubular structure with a diameter varying gradually. A proximal end of the second transition tube body 15 is connected to the middle tube body 12, and a distal end of the second transition tube body 15 is connected to the distal tube body 13. Accordingly, the proximal diameter of the second transition tube body 15 is smaller than its distal diameter thereof.

By means of the first transition tube body 14 and the second transition tube body 15, smooth transitional connections are formed respectively between the middle tube body 12 and the proximal tube body 11 and between the middle tube body 12 and the distal tube body 13, avoiding the risk of endoleak or local embolism caused by a locally formed groove-like dead structure due to the abrupt change of the shape.

Each of the tube bodies 11, 12, 13, 14, and 15 of the stent body 1 is comprised of one or more support frames 101 and the covering membrane 102 supported by the one or more support frames 101.

Particularly, the support frames 101 in the first transition tube body 14 and the second transition tube body 15 are defined as transition support frames 104. The transition support frames 104 are tapered, to maintain the truncated cone structures of the first transition tube body 14 and the second transition tube body 15. A smaller end surface of the transition support frame 104 faces the middle tube body 12.

In this embodiment, each of the first transition tube body 14 and the second transition tube body 15 is provided with only one transition support frame 104. It can be understood that the number of the transition support frame 104 is not limited. If it is need, multiple transition support frames 104 can be used, the multiple transition support frames 104 are tapered, and located on the same frustum surface.

Respective support frames 101 of the proximal tube body 11, the middle tube body 12 and the distal tube body 13 are cylindrical, to maintain respective structures of a constant diameter of each of the tube bodies 11, 12, and 13. A plurality of support frames 101 are provided at intervals in each of the tube bodies 11, 12, and 13, which gives the tube bodies 11, 12, and 13 all a good flexibility.

Preferably, the proximal tube body 11 is also provided with a last-release portion 105, to release the proximal end of the covered stent 100 at the end of deployment. The last-releasing portion 105 is a radially collapsible ring structure, fixed to the covering membrane 102, and extended proximally beyond a proximal edge of the covering membrane 102. The structure of the last-releasing portion 105 may be the same as the structure of the support frame 101, but only part of the last-releasing portion 105 is attached to the covering membrane 102.

Further, the last-releasing portion 105 is further preferably provided with protruding barbs arranged circumferentially (not shown). A plurality of barbs may be provided. After the covered stent 100 is released, the barbs may hook up to the wall of blood vessel of a human body to increase the stability of the released covered stent 100.

A plurality of radiopaque markers 106 are provided at a proximal end of the covering membrane 102 adj acent to the edge, for the purpose of well observing the position of the covered stent 100 where it is released during the operation. In this embodiment, the plurality of radiopaque markers 106 are provided, which are arranged annually or along an 8-shaped structure, and fixed to the covering membrane 102 by suturing or hot-pressing. The radiopaque marker 106 is made of a radiopaque material, including but not limited to, gold, platinum, palladium, rhodium, tantalum, or alloys or composites of them. Taking platinum as an example, its alloy may be platinum-tungsten, platinum-iridium and the like.

Referring to FIGs. 1 to 3, the outer branch 2 protrudes outside of the stent body 1, and has one end connected to a side wall of the middle tube body 12, with an inner opening 21 defined on the side wall of the middle tube body 12. The outer branch 2 extends from the inner opening 21 in a direction away from the middle tube body 12.

The outer branch 2 may be a tubular structure with a unified diameter or variable diameter. In this embodiment, the outer branch 2 includes a tubular membrane 22 and a plurality of support rings 23 fixed to the surface of the membrane 22. The plurality of support rings 23 are arranged at intervals along an axial direction of the outer branch 2. Each support ring 23 may have the same structure as the support frame 101 of the stent body 1, and is also a radially collapsible and expandable ring structure. The membrane 22 can be formed of any suitable covering membrane material, including but not limited to, low-porosity woven or knitted polyester, terylene materials, expanded polytetrafluoroethylene, polyurethane, silicone, ultra-high-molecular-weight polyethylene or other suitable materials.

In other embodiments which are not shown, the outer branch 2 may be in the form of a bare stent, for example, a mesh-like bare stent.

The end of the outer branch 2 provided with the inner opening 21 is fixed to the covering membrane 102 of the middle tube body 12 by suturing, and is integrated with the middle tube body 12. The inner lumen of the outer branch 2 communicates with the inner lumen of the middle tube body 12. In practical production, a through hole is made in the covering membrane 102 of the middle tube body 12, and the outer branch 2 is sutured around the through hole correspondingly.

The length L2 of the outer branch 2 is preferably 5 to 20 mm. When the covered stent 100 is released to a site requiring treatment, the outer branch 2 can be released into innominate artery. Since the innominate artery is located at the leftmost end (i.e., the proximal-most end) of the aortic arch, the position of the covered stent 100 can be well stabilized by engagement of the outer branch 2 into the innominate artery, so that the covered stent 100 as a whole is unlikely to move.

The outer branch 2 is preferably further provided with a marker 24. As shown in FIG. 3, the marker 24 is dot-like, and a plurality of markers are provided at intervals along the extension direction of the outer branch 2. Further, an outer periphery of the inner opening 21 may also be provided with a plurality of markers 24. The markers 24 are made of radiopaque material, and are fixed to the membrane 22 by suturing or hot-pressing. With these markers 24, the position of the outer branch 2 can be visualized during operation, so that an external branch stent can be more quickly inserted into the innominate artery through the outer branch 2.

Referring to FIGs. 1 to 3 again, the first inner branch 3 is generally located inside the stent body 1, and attached to an inner wall of the middle tube body 12. The first inner branch 3 has one end connected to the side wall of the middle tube body 12, with a first outer opening 31 defined on the side wall of the middle tube body 12. The first outer opening 31 is closer to the distal end than the inner opening 21 of the outer branch 2.

Similar to the outer branch 2, the first inner branch 3 is a tubular structure with a constant diameter or variable diameter, and may be a bare stent or a stent with a membrane, which is not described in detail here. Similarly, a through hole is made in the covering membrane 102 of the middle tube body 12 corresponding to the first inner branch 3, and the first outer opening 31 of the first inner branch 3 is sutured to the covering membrane 102.

Except for the first outer opening 31, the remaining portion of the first inner branch 3 is located inside the middle tube body 12, and fixed to the wall of the middle tube body 12 from inside by suturing or bonding.

Preferably, the first inner branch 3 extends from the first outer opening 31 towards the proximal end along the wall of the middle tube body 12. In this embodiment, as shown in FIG. 1, the extension direction of the first inner branch 3 is substantially consistent with the axis L of the stent body 1, and the axis of the first inner branch 3 is in the same plane as the axis L of the stent body 1.

Preferably, the first inner branch 3 is also provided with a plurality of markers 32 along its extension direction. Further, an outer periphery of the first outer opening 31 may also be provided with a plurality of markers 32. The related features of the marker 32 are similar to those of the marker24 on the outer branch 2, and will not be described here again.

Referring to FIGs. 1 to 3 again, the second inner branch 4 is closer to the distal end than the first inner branch 3, and the second inner branch 4 is also generally located inside the middle tube body 12 and attached to the wall of the middle tube body 12 from inside. The second inner branch 4 has a second outer opening 41 on the side wall of the middle tube body 12, and the second outer opening 41 is closer to the distal end than the first outer opening 31.

In this embodiment, the second inner branch 4 extends from the second outer opening 41 towards the distal end along the wall of the middle tube body 12. The extension direction of the second inner branch 4 is opposite to the extension direction of the first inner branch 3, to avoid a situation where the blood flow entrance is blocked by the first inner branch 3 and thus the blood flow cannot enter.

The second inner branch 4 is also provided with a plurality of markers 42 along its extension direction. Further, an outer periphery of the second outer opening 41 may also be provided with a plurality of markers 42. Other features of the second inner branch 4 may be referred to as those of the first inner branch 3, and will not be repeated here again.

As shown in FIG. 1, the inner opening 21, the first outer opening 31 and the second outer opening 41 are substantially arranged at intervals on a same line parallel to the axis L of stent body 1. It can be understood that there are enough spaces among the three openings circumferentially.

Referring to FIGs. 1 to 3, in this embodiment, the first inner branch 3 and the second inner branch are substantially symmetrically arranged.

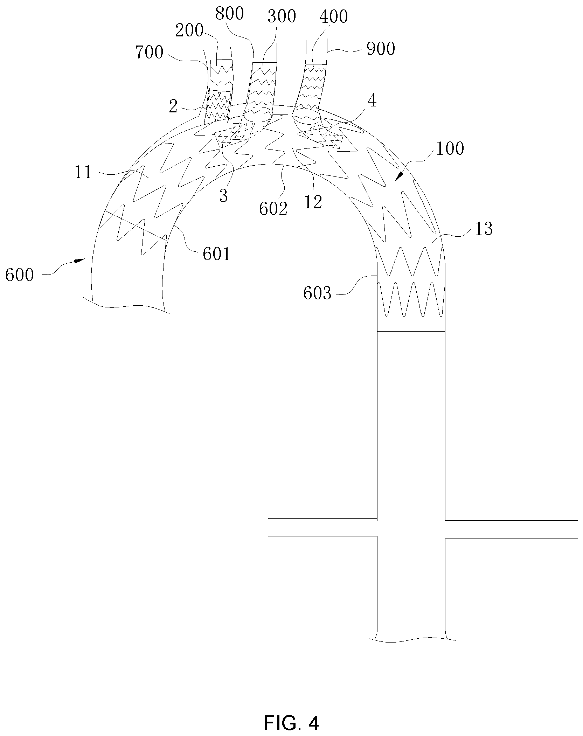

On the basis of the foregoing descriptions of the structure of the covered stent 100, and referring to FIG. 4, the covered stent 100 is delivered into the aorta 600 by a delivery device during use to reconstruct the blood flow channels in the aorta 600. The proximal tube body 11 of the stent body 1 extends into the ascending aorta 601 of the aorta 600, which is attached against to the wall of the ascending aorta 601. The distal tube body 13 is positioned in the descending aorta 603 of the aorta 600 and is attached to the vascular wall of the descending aorta 603. The middle tube body 12 is located at the aortic arch 602 of the aorta 600. The outer branch 2 extends into an innominate artery 700, to stabilize the position of the covered stent 100 in the aorta 600, so that the covered stent 100 as a whole is unlikely to move.

For the three branch blood vessels of the aortic arch 600, appropriate external branch stents can be selected respectively, and then inserted into corresponding branch blood vessels via the covered stent 100.

Specifically, a first branch stent 200 extends out of the outer branch 2 and into the innominate artery 700, to reconstruct the blood flow channel between aorta 600 and innominate artery 700. A second branch stent 300 extends out of the first inner branch 3 through the first outer opening 31, and then into the left common carotid artery 800, to reconstruct the blood flow channel between the aorta 600 and the left common carotid artery 800. A third branch stent 400 extends out of the second inner branch 4 through the second outer opening 41, and then into the left subclavian artery 900, to reconstruct the blood flow channel between aorta 600 and the left subclavian artery 900.

The exemplary implantation process of the covered stent 100 and the subsequent branch stents 200, 300, and 400 are described substantial as follows:
1. Releasing the proximal tube body 11 of the covered stent 100 first;
2. Then releasing the proximal portion of the middle tube body 12, wherein a pre-placed guidewire was previously inserted in the outer branch 2. When the pre-placed guidewire in the outer branch 2 is released, the guidewire is delivered by a snaring device or another device into the innominate artery 700, to deliver the outer branch 2 into the innominate artery 700;
3. Releasing the remaining portions of the stent body 1 rapidly, and finishing the last release of the last-releasing portion 105 of the proximal tube body 11, to anchor the covered stent 100 in aorta 600.
4. Reconstructing the three branch blood vessels including the innominate artery 700, the left common carotid artery 800, and the left subclavian artery 900 respectively in sequence by guidewire and catheter exchange technique; releasing the branch stents 200, 300, and 400 of corresponding dimensions respectively. Specifically, the first branch stent 200 is released in the outer branch 2 to reconstruct the innominate artery 700, the second branch stent 300 is released in the first inner branch 3 to reconstruct the left common carotid artery 800, and the third branch stent 400 is released in the second inner branch 4 to reconstruct the left subclavian artery 900.

As described above, in the covered stent 100 of this embodiment, the outer branch 2, the first inner branch 3 and the second inner branch 4 are integrally formed with the stent body 1. The branches 2, 3, and 4 can provide corresponding positioning and supporting functions to the three external branch stents 200, 300, and 400 respectively. Further, the branches 2, 3, and 4 are located at the middle tube body 12 which has a smaller diameter of stent body 1. Due to the formation of the concave portion 103 at the middle tube body 12, a space formed between the concave portion 103 and the vascular wall of the aortic arch 602 allows to provide enough room to manipulating the guidewire and the catheter during the endovascular therapy. Therefore, the three external branch stents 200, 300, and 400 can be quickly delivered into branch arteries during the operation for reconstruction of the three main branches of arch of aorta 600. Moreover, due to the smaller diameter of the middle tube body 12, the covered stent 100 will not compress the branch vessels to cause the occlusion of the branch vessels after the branch stents 200, 300, and 400 have been implanted into the blood vessels. The branch stents 200, 300, and 400 are respectively anchored to the covered stent 100 by the outer branch 2, the first inner branch 3 and the second inner branch 4. The surgical operations are simpler, and the surgical procedure is shortened.

Particularly, among the branches 2, 3, and 4 of the covered stent 100, the most proximal outer branch 2 protrudes outside of the stent body 1. When the covered stent 100 is delivered to a preplanned site in aorta 600, the outer branch 2 can extend into the innominate artery 700, facilitating the determination of whether the covered stent 100 has been delivered in place. Following the deployments of the outer branch 2, the engagement of the outer branch 2 with the innominate artery 700, and the engagement of the stent body 1 with the aorta 600 will make the covered stent 100 as a whole unlikely to move. This facilitates the subsequent implantation of the branch stents. The two inner branches 3 and 4 closer to the distal end of the outer branch 2 are located inside the stent body 1, and the two inner branches 3, 4 do not extend out of the stent body 1, just with their outer openings 31, 41 exposed on the side wall of the stent body 1. With the operation space provided by the concave portion 103, larger position adjustment spaces are provided for branch stents insertion into the branch arteries via the inner branches 3 and 4, so that the branch stents can be more conveniently implanted into the branch artery vessels, with no requirement to exactly align the openings of the inner branches 3, 4 with the orifices of the branch vessels. Therefore, the covered stent 100 has more widespread applicability and higher universality.

In addition, in this embodiment, the first inner branch 3 relatively close to the outer branch 2 is an antegrade inner branch, in which the direction of blood flow is consistent with the direction of blood flow in the aorta 600, so embolism is not likely to occur.

Further, the extension direction of the second inner branch 4 is opposite to the extension direction of the first inner branch 3, to avoid a situation where the blood flow entrance is blocked by the first inner branch 3 and thus the blood cannot enter. Because the second inner branch 4 is close to the descending aorta 603 side during use, and the extension direction of the second inner branch 4 is consistent with the extension direction of the descending aorta 603, the extension of the second inner branch 4 towards the distal end does not affect the flow of blood into the second inner branch 4.

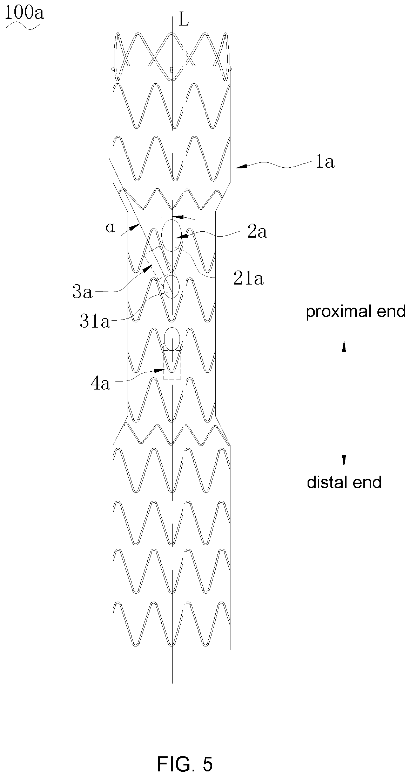

Referring to FIG. 5, which shows the structure of a covered stent 100a according to a second embodiment of the present invention.

The structure of the covered stent 100a of this embodiment is similar to the covered stent 100 of the first embodiment. For the same parts, references may be made to the above descriptions of the first embodiment, and the details will not be repeated here. This embodiment differs from the first embodiment in the extension direction of the first inner branch 3a.

As shown in FIG. 5, in this embodiment, the first inner branch 3a also extends from the first outer opening 31a towards the proximal end, but with the extension towards the proximal end, the first inner branch 3a further extends in a circumferential direction of the stent body 1a from the second outer opening 31a. That is, the extension direction of the first inner branch 3a is inclined relative to the extension direction of the stent body 1a.

Specifically, in this embodiment, the extension form of the first inner branch 3a in the stent body 1a may be substantially considered as the shape of a short segment of a spiral line. An angle α is defined between the axis of the first inner branch 3a and the axis L of the stent body 1a, and the angle α is greater than 0 degrees and less than 90 degrees.

Due to the extension manner of the first inner branch 3a, the first inner branch 3a will not block the inner opening 21a of the outer branch 2a even extending by a long length, so that the blood in the aorta can smoothly enter the outer branch 2a through the inner opening 21a and then enter the innominate artery. Moreover, when a branch stent is implanted in the outer branch 2a, the branch stent would extend towards the inner wall of the stent body 1a for a certain length to form an anchoring area. The arrangement of the first inner branch 3a extending inclinedly relative to the axis L of the stent body 1a can prevent interferences between the branch stents implanted in the outer branch 2a and the first inner branch 3a.

The configuration and structures of the stent body 1a, the outer branch 2a and the second inner branch 4a in the covered stent 100a of this embodiment are the same as those in the first embodiment, and the details will not be repeated here.

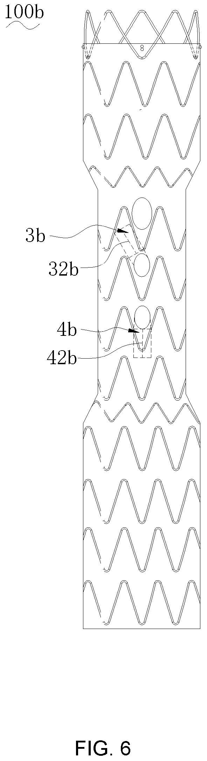

Referring to FIG. 6, which shows the structure of a covered stent 100b according to a third embodiment of the present invention.

The covered stent 100b of this embodiment is similar to the covered stent 100a of the second embodiment. For the same parts, references may be made to the above descriptions of the second embodiment and the first embodiment, and the details will not be repeated here. This embodiment differs from the second embodiment in that the marker 32b of the first inner branch 3b and the marker 42b of the second inner branch 4b are both strip-shaped.

As shown in FIG. 6, the first inner branch 3b is provided with a plurality of markers 32b, each of the markers 32b is strip-shaped, and the plurality of markers 32b are arranged in sequence along an axial direction of the first inner branch 3b, and arranged linearly as a whole. The extension direction of the first markers 32b is consistent with the overall extension direction of the multiple markers 32b. Specifically, a single marker 32b may be formed by a continuous radiopaque wire, or continuously wound on the metal frame of the first inner branch 3b.

The second inner branch 4b is provided with a plurality of markers 42b. Similarly, the first markers 42b is strip-shaped, and the plurality of markers 42b are arranged in sequence along an axial direction of the second inner branch 4b, and arranged linearly as a whole. The extension direction of each of the marker 42b is consistent with the overall extension direction of the multiple markers 42b. Specifically, a single marker 42b may be formed by a continuous radiopaque wire, or continuously wound on the metal frame of the second inner branch 4b.

Compared with the dot-like markers 32, 42 in the first embodiment, the strip-shaped markers 32b, 42b in this embodiment are continuous. In the case where a plurality of markers are arranged with intervals along the axial direction of the inner branches 3b, 4b, the overall positions of the first inner branch 3b and the second inner branch 4b can be visualized more easily through the strip-shaped markers extending along the axial direction of the inner branches.

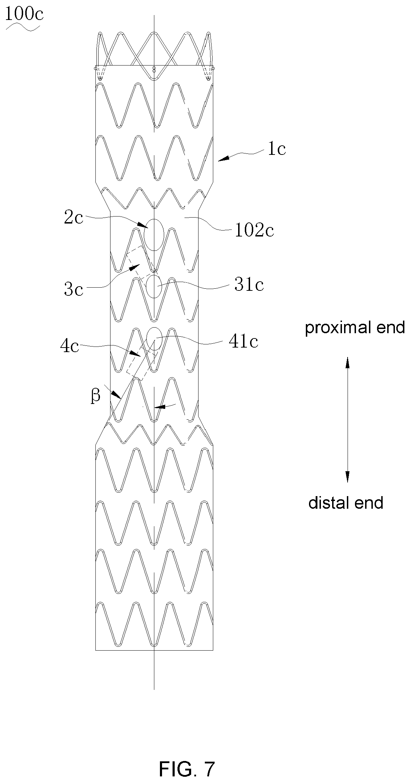

Referring to FIG. 7, which shows the structure of a covered stent 100c according to a fourth embodiment of the present invention.

The covered stent 100c of this embodiment is similar to the covered stent 100a of the second embodiment, but differs from the second embodiment in the extension direction of the second inner branch 4c.

As shown in FIG. 7, the second inner branch 4c also extends from the second outer opening 41c towards the distal end, however, with extension towards the distal end, the second inner branch 4c further extends in a circumferential direction of the stent body 1c from the second outer opening 41c. That is, the extension direction of the second inner branch 4c is inclined relative to the extension direction of the stent body 1c.

Specifically, in this embodiment, the extension form of the second inner branch 4c in the stent body 1c may also be substantially considered as the shape of a short segment of spiral line. An angle β is defined between the axis of the second inner branch 4c and the axis L of the stent body 1c, and the angle β is greater than 0 degrees and less than 90 degrees.

Because of the extension manner of the second inner branch 4c, when the covered stent 100c is implanted into aorta, the second inner branch 4c can be located in the arch bending area of the aortic arch. The design of the second inner branch 4c extending inclinedly relative to the axis L of the stent body 1c can avoid folding area of the covering membrane 102c due to bending, thereby preventing the entrance from being blocked by the covering membrane 102c.

In the structure shown in FIG. 7, a circumferential extension direction of the second inner branch 4c is the same as a circumferential extension direction of the first inner branch 3c. The second inner branch 4c and the first inner branch 3c are located on the same side of a line connecting the second outer opening 41c and the first outer opening 31c. In other structures not shown, the circumferential extension direction of the second inner branch 4c may be opposite to the circumferential extension direction of the first inner branch 3c.

The arrangements and structures of the stent body 1c, the outer branch 2c and the first inner branch 3c in the covered stent 100c of this embodiment are the same as those of the second embodiment, and the details will not be repeated here.

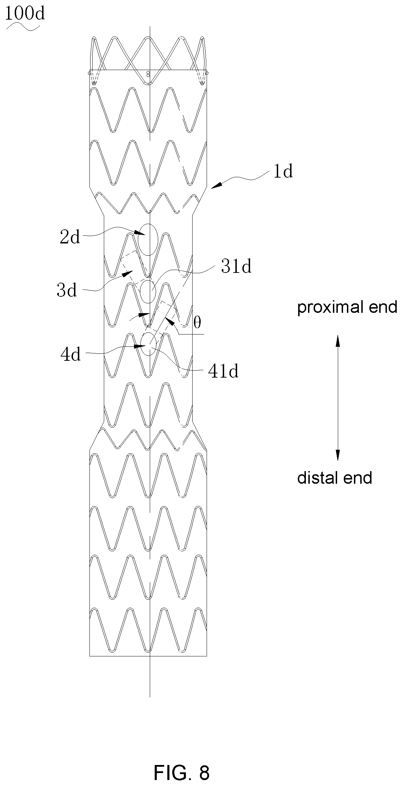

Referring to FIG. 8, which shows the structure of a covered stent 100d according to a fifth embodiment of the present invention.

The covered stent 100d of this embodiment is similar to the covered stent 100a of the second embodiment, and differs from the second embodiment in the extension direction of the second inner branch 4d.

As shown in FIG. 8, in this embodiment, the second inner branch 4d extends from the second outer opening 41d towards the proximal end, however, with extension towards the proximal end, the second inner branch 4d further extends in a circumferential direction of the stent body 1d from the second outer opening 41d. The extension direction of the second inner branch 4d is inclined relative to the extension direction of the stent body 1d.

Specifically, in this embodiment, the extension fashion of the second inner branch 4d in the stent body 1d may also be substantially considered as the shape of a short segment of spiral line. An angle θ is defined between the axis of the second inner branch 4d and the axis L of the stent body 1d, and the angle θ is greater than 0 degree and less than 90 degrees.

A circumferential extension direction of the second inner branch 4d is opposite to a circumferential extension direction of the first inner branch 3d. The second inner branch 4d and the first inner branch 3d are respectively located on two sides of a line connecting the first outer opening 31d and the second outer opening 41d. Taking the viewing direction in FIG. 8 as a reference, the first inner branch 3d extends leftwards, and the second inner branch 4d extends rightwards.

In this embodiment, the second inner branch 4d is also an antegrade branch, which is adapted to the direction of blood flow direction. The second inner branch 4d and the first inner branch 3d both extend inclinedly, and their inclination directions are opposite, such that interferences between these two branches are avoided.

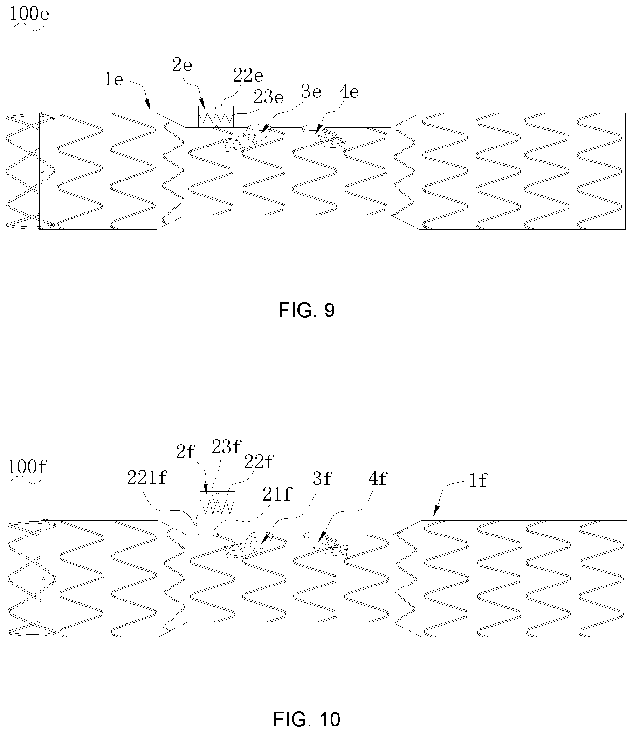

Referring to FIG. 9, which shows the structure of a covered stent 100e according to a sixth embodiment of the present invention.

The covered stent 100e of this embodiment is similar to the covered stent 100 of the first embodiment, but differs from the first embodiment in that the length of the outer branch 2e is shorter, which is about 1/3 to 1/2 of the length of the outer branch 2 in the first embodiment. Only one support ring 23e is provided on the membrane 22e of the outer branch 2e.

In this embodiment, a shorter outer branch 2e is provided, having increased flexibility for implantation, and thus can be adapted to patients with different anatomical structures. In this case, the shorter outer branch 2e may serve as a fixing end for the external branch stent, and the position of the innominate artery is not strictly limited.

The arrangements and structures of the stent body 1e, the first inner branch 3e and the second inner branch 4e in the covered stent 100e of this embodiment are the same as those of the first embodiment, and the details will not be repeated here.

Referring to FIG. 10, which shows the structure of a covered stent 100f according to a seventh embodiment of the present invention.

The covered stent 100f of this embodiment is similar to the covered stent 100 of the first embodiment, but differs from the first embodiment in the structure of the outer branch 2f.

As shown in FIG. 10, in this embodiment, the outer branch 2f has a flexible section 221f capable of flexible deformation, and the flexible section 221f extends outwards from the inner opening 21f. The flexible section 221 is made of a flexible membrane material on which no supporting structure is arranged, so as to provide greater flexibility, and is adapted to more patients with different anatomical structures. The length of the flexible section 221 is substantially 2 mm to 10 mm, and does not exceed 1/2 of the total length of the outer branch 2f.

The flexible section 221f may be formed by a section of the membrane 22f adjacent to the stent body 1f without support ring 23f provided thereon. That is, the support ring 23f is not arranged in a section of the membrane 22f of the outer branch 2f with a length ranging from about 2 mm to 10 mm from the inner opening 21f. The section of the membrane 22f without support ring 23f provided thereon constitutes the flexible section 221f. The remaining part of the membrane 22f other than the flexible section 221f is provided with the support ring 23f to maintain the outer branch 2f tubular.

The arrangements and structures of the stent body 1f, the first inner branch 3f and the second inner branch 4f in the covered stent 100f of this embodiment are the same as those of the first embodiment, and the details will not be repeated here.

In the above embodiments, all the examples with two inner branches provided on the stent body are taken for illustration, and in combination with the arrangement of the outer branch, the three branch artery vessels of the aorta can be reconstructed. However, it can be understood that in some embodiments not shown, the number of inner branches may be decreased to one, for the cases in which two branch arteries need to be reconstructed.

As can be known from the detailed descriptions of the above embodiments, by using the covered stent of the present invention, the aortic stent and the external branch artery stents may be independent structures, to adapt to various normal and abnormal blood vessels. Aortic vascular stent and branch artery vascular stent of appropriate sizes can be selected according to the specific conditions of the lesion site to form a vascular stent system that is most suitable for the patient, avoiding the customization of stents. The covered stents of the present invention are suitable for mass production, which can save time, and is easy to operate.

## Claims

1. A covered stent, comprising:
a stent body (1, 1a, 1c, 1d, 1e, 1f) being of a tubular structure with a covering membrane (102) on a surface thereof, and forming a radially recessed concave portion (103) thereon;
an outer branch (2, 2a, 2c, 2d, 2e, 2f) extending outside the stent body (1, 1a, 1c, 1d, 1e, 1f), having one end connected to a side wall of the concave portion (103) with an inner opening (21, 21a, 21f) on the side wall of the concave portion (103); and
at least one inner branch (3, 3a, 3b, 3c, 3d, 3e, 3f, 4, 4a, 4b, 4c, 4d, 4e, 4f), attached to an inside wall of the stent body (1, 1a, 1c, 1d, 1e, 1f), each of the at least one inner branch (3, 3a, 3b, 3c, 3d, 3e, 3f, 4, 4a, 4b, 4c, 4d, 4e, 4f) having one end connected to the side wall of the concave portion (103) and with an outer opening (31, 31a, 31c, 31d, 41, 41c, 41d) on the side wall of the concave portion (103),
**characterized in that** the stent body (1, 1a, 1c, 1d, 1e, 1f) comprises a proximal tube body (11), a middle tube body (12), and a distal tube body (13) in sequence from a proximal end to the distal end; each of the proximal tube body (11), the middle tube body (12) and the distal tube body (13) is a tubular structure with a substantially constant diameter, a diameter of the middle tube body (12) is less than a diameter of the proximal tube body (11) and a diameter of the distal tube body (13), and the middle tube body (12) constitutes the concave portion (103),
wherein two ends of the middle tube body (12) are respectively connected to the proximal tube body (11) and the distal tube body (13) by two transition tube bodies (14, 15); each of the transition tube bodies (14, 15) has a tapered transition support frame (104) for supporting the covering membrane (102), and a smaller end of the transition support frame (104) faces the middle tube body (12),
wherein the one end of the outer branch (2, 2a, 2c, 2d, 2e, 2f) is connected to a side wall of the middle tube body (12), with the inner opening (21, 21a, 21f) defined on the side wall of the middle tube body (12), and the one end of each inner branch (3, 3a, 3b, 3c, 3d, 3e, 3f, 4, 4a, 4b, 4c, 4d, 4e, 4f) is connected to the side wall of the middle tube body (12), with the outer opening (31, 31a, 31c, 31d, 41, 41c, 41d) defined on the side wall of the middle tube body (12),
wherein the outer opening (31, 31a, 31c, 31d, 41, 41c, 41d) is closer to a distal end than the inner opening (21, 21a, 21f).

2. The covered stent according to claim 1, wherein at least one inner branch (3, 4) comprises two inner branches (3, 3a, 3b, 3c, 3d, 3e, 3f, 4, 4a, 4b, 4c, 4d, 4e, 4f), which are a first inner branch (3, 3a, 3b, 3c, 3d, 3e, 3f) and a second inner branch (4, 4a, 4b, 4c, 4d, 4e, 4f) respectively, wherein the first inner branch (3, 3a, 3b, 3c, 3d, 3e, 3f) has a first outer opening (31, 31a, 31c, 31d) on the side wall of the concave portion (103), the second inner branch (4, 4a, 4b, 4c, 4d, 4e, 4f) has a second outer opening (41, 41c, 41d) on the side wall of the concave portion (103), and the second outer opening (41, 41c, 41d) is closer to the distal end than the first outer opening (31, 31a, 31c, 31d).

3. The covered stent according to claim 2, wherein the first inner branch (3, 3a, 3b, 3c, 3d, 3e, 3f) extends from the first outer opening (31, 31a, 31c, 31d) towards a proximal end.

4. The covered stent according to claim 3, wherein the covered stent ((1, 1a, 1c, 1d, 1e, 1f)) has an additional feature a) or b):
a) the second inner branch (4, 4a, 4b, 4c, 4e, 4f) extends from the second outer opening (41, 41c) towards the distal end;
b) with extension towards the proximal end, the first inner branch (3a, 3b, 3c, 3d) further extends in the circumferential direction of the stent body from the first outer opening (31a, 31c, 31d).

5. The covered stent according to claim 4 with feature a), wherein with extension towards the distal end, the second inner branch (4c) further extends in a circumferential direction of the stent body from the second outer opening (41c).

6. The covered stent according to claim 4 with feature b), wherein the second inner branch (4d) extends from the second outer opening (41d) towards the proximal end, and with extension towards the proximal end, the second inner branch (4d) further extends in the circumferential direction of the stent body (1d) from the second outer opening (41d); and a circumferential extension direction of the second inner branch (4d) is opposite to a circumferential extension direction of the first inner branch (3d).

7. The covered stent according to any one of claims 1 to 6, wherein a length of the outer branch (2, 2a, 2c, 2d, 2e, 2f) is 5 mm to 20 mm.

8. The covered stent according to any one of claims 1 to 7, wherein the outer branch (2f) has a flexible section (221f) capable of flexible deformation, and the flexible section (221f) extends outwards from the inner opening (21f).

9. The covered stent according to claim 8, wherein the covered stent has an additional feature c) or d):
c) the flexible section (221f) is made of flexible membrane material;
d) a length of the flexible section (221f) is 2 mm to 10 mm, without exceeding 1/2 of the total length of the outer branch (2f).

10. The covered stent according to any one of claims 1 to 9, wherein each of the outer branch (2) and the inner branch (3, 3b. 4, 4b) is with a marker (24, 32, 32b, 42, 42b), and the marker (24, 32, 32b, 42, 42b) is made of radiopaque material.

11. The covered stent according to claim 10, wherein the covered stent has an additional feature e), f), or g):
e) the marker is circumferentially arranged at the inner opening or the outer opening;
f) the outer branch or the at least one inner branch (3b, 4b) is provided with the marker (32b, 42b) along an extension direction thereof;
g) the marker is hot pressed or sutured on the outer branch (2) and the inner branch (3, 32, 4, 42).

12. The covered stent according to claim 1, wherein the covered stent has an additional feature n):
n) the proximal tube body (11) is provided with a last-releasing portion (105) that is a radially collapsible ring structure fixed to the covering membrane (102), and extending proximally beyond a proximal edge of the covering membrane (102).

13. The covered stent according to claim 12, wherein the last-releasing portion (105) is provided with protruding barbs arranged circumferentially.

14. The covered stent according to any one of claims 1 to 13, wherein a radiopaque marker (106) is provided at an end of the covering membrane (102), and the radiopaque marker (106) is made of radiopaque material.

## Patentansprüche

1. Ummantelter Stent, umfassend:
einen Stentkörper (1, 1a, 1c, 1d, 1e, 1f), der eine röhrenförmige Struktur mit einer Abdeckmembran (102) auf einer Oberfläche davon aufweist und einen radial ausgesparten konkaven Abschnitt (103) darauf bildet;
einen äußeren Zweig (2, 2a, 2c, 2d, 2e, 2f), der sich außerhalb des Stentkörpers (1, 1a, 1c, 1d, 1e, 1f) erstreckt, wobei ein Ende mit einer Seitenwand des konkaven Abschnitts (103) mit einer inneren Öffnung (21, 21a, 21f) auf der Seitenwand des konkaven Abschnitts (103) verbunden ist; und
mindestens einen inneren Zweig (3, 3a, 3b, 3c, 3d, 3e, 3f, 4, 4a, 4b, 4c, 4d, 4e, 4f), der an einer Innenwand des Stentkörpers (1, 1a, 1c, 1d, 1e, 1f) befestigt ist, wobei jeder des mindestens einen inneren Zweigs (3, 3a, 3b, 3c, 3d, 3e, 3f, 4, 4a, 4b, 4c, 4d, 4e, 4f) ein Ende aufweist, das mit der Seitenwand des konkaven Abschnitts (103) und mit einer äußeren Öffnung (31, 31a, 31c, 31d, 41, 41c, 41d) auf der Seitenwand des konkaven Abschnitts (103) verbunden ist,
**dadurch gekennzeichnet, dass** der Stentkörper (1, 1a, 1c, 1d, 1e, 1f) einen proximalen Röhrenkörper (11), einen mittleren Röhrenkörper (12) und einen distalen Röhrenkörper (13) der Reihe nach von einem proximalen Ende zu dem distalen Ende umfasst; wobei jeder des proximalen Röhrenkörpers (11), des mittleren Röhrenkörpers (12) und des distalen Röhrenkörpers (13) eine röhrenförmige Struktur mit einem im Wesentlichen konstanten Durchmesser ist, ein Durchmesser des mittleren Röhrenkörpers (12) kleiner als ein Durchmesser des proximalen Röhrenkörpers (11) und ein Durchmesser des distalen Röhrenkörpers (13) ist und der mittlere Röhrenkörper (12) den konkaven Abschnitt (103) bildet,
wobei zwei Enden des mittleren Röhrenkörpers (12) jeweils mit dem proximalen Röhrenkörper (11) und dem distalen Röhrenkörper (13) durch zwei Übergangsröhrenkörper (14, 15) verbunden sind; wobei jeder der Übergangsröhrenkörper (14, 15) einen sich verjüngenden Übergangsstützrahmen (104) zum Stützen der Abdeckmembran (102) aufweist und ein kleineres Ende des Übergangsstützrahmens (104) dem mittleren Röhrenkörper (12) zugewandt ist,
wobei das eine Ende des äußeren Zweigs (2, 2a, 2c, 2d, 2e, 2f) mit einer Seitenwand des mittleren Röhrenkörpers (12) verbunden ist, wobei die innere Öffnung (21, 21a, 21f) auf der Seitenwand des mittleren Röhrenkörpers (12) definiert ist, und das eine Ende jedes inneren Zweigs (3, 3a, 3b, 3c, 3d, 3e, 3f, 4, 4a, 4b, 4c, 4d, 4e, 4f) mit der Seitenwand des mittleren Röhrenkörpers (12) verbunden ist, wobei die äußere Öffnung (31, 31a, 31c, 31d, 41, 41c, 41d) auf der Seitenwand des mittleren Röhrenkörpers (12) definiert ist,
wobei die äußere Öffnung (31, 31a, 31c, 31d, 41, 41c, 41d) näher an einem distalen Ende als die innere Öffnung (21, 21a, 21f) ist.

2. Ummantelter Stent nach Anspruch 1, wobei mindestens ein innerer Zweig (3, 4) zwei innere Zweige (3, 3a, 3b, 3c, 3d, 3e, 3f, 4, 4a, 4b, 4c, 4d, 4e, 4f) umfasst, die jeweils ein erster innerer Zweig (3, 3a, 3b, 3c, 3d, 3e, 3f) und ein zweiter innerer Zweig (4, 4a, 4b, 4c, 4d, 4e, 4f) sind, wobei der erste innere Zweig (3, 3a, 3b, 3c, 3d, 3e, 3f) eine erste äußere Öffnung (31, 31a, 31c, 31d) auf der Seitenwand des konkaven Abschnitts (103) aufweist, der zweite innere Zweig (4, 4a, 4b, 4c, 4d, 4e, 4f) eine zweite äußere Öffnung (41, 41c, 41d) auf der Seitenwand des konkaven Abschnitts (103) aufweist und die zweite äußere Öffnung (41, 41c, 41d) näher an dem distalen Ende als die erste äußere Öffnung (31, 31a, 31c, 31d) ist.

3. Ummantelter Stent nach Anspruch 2, wobei sich der erste innere Zweig (3, 3a, 3b, 3c, 3d, 3e, 3 f) von der ersten äußeren Öffnung (31, 31a, 31c, 31d) zu einem proximalen Ende erstreckt.

4. Ummantelter Stent nach Anspruch 3, wobei der ummantelte Stent ((1, 1a, 1c, 1d, 1e, 1f)) ein zusätzliches Merkmal a) oder b) aufweist:
a) der zweite innere Zweig (4, 4a, 4b, 4c, 4e, 4f) erstreckt sich von der zweiten äußeren Öffnung (41, 41c) zu dem distalen Ende;
b) mit Erstreckung zu dem proximalen Ende erstreckt sich der erste innere Zweig (3a, 3b, 3c, 3d) weiter in der Umfangsrichtung des Stentkörpers von der ersten äußeren Öffnung (31a, 31c, 31d).

5. Ummantelter Stent nach Anspruch 4 mit Merkmal a), wobei sich mit Erstreckung zu dem distalen Ende der zweite innere Zweig (4c) weiter in einer Umfangsrichtung des Stentkörpers von der zweiten äußeren Öffnung (41c) erstreckt.

6. Ummantelter Stent nach Anspruch 4 mit Merkmal b), wobei sich der zweite innere Zweig (4d) von der zweiten äußeren Öffnung (41d) zu dem proximalen Ende erstreckt, und mit Erstreckung zu dem proximalen Ende erstreckt sich der zweite innere Zweig (4d) weiter in der Umfangsrichtung des Stentkörpers (1d) von der zweiten äußeren Öffnung (41d); und eine Umfangserstreckungsrichtung des zweiten inneren Zweigs (4d) entgegengesetzt zu einer Umfangserstreckungsrichtung des ersten inneren Zweigs (3d) ist.

7. Ummantelter Stent nach einem der Ansprüche 1 bis 6, wobei eine Länge des äußeren Zweigs (2, 2a, 2c, 2d, 2e, 2f) 5 mm bis 20 mm beträgt.

8. Ummantelter Stent nach einem der Ansprüche 1 bis 7, wobei der äußere Zweig (2f) einen flexiblen Abschnitt (221f) aufweist, der zu einer flexiblen Verformung fähig ist, und sich der flexible Abschnitt (221f) von der inneren Öffnung (21f) nach außen erstreckt.

9. Ummantelter Stent nach Anspruch 8, wobei der ummantelte Stent ein zusätzliches Merkmal c) oder d) aufweist:
c) der flexible Abschnitt (221f) ist aus flexiblem Membranmaterial hergestellt;
d) eine Länge des flexiblen Abschnitts (221f) beträgt 2 mm bis 10 mm, ohne 1/2 der Gesamtlänge des äußeren Zweigs (2f) zu überschreiten.

10. Ummantelter Stent nach einem der Ansprüche 1 bis 9, wobei jeder von dem äußeren Zweig (2) und dem inneren Zweig (3, 3b, 4, 4b) mit einer Markierung (24, 32, 32b, 42, 42b) versehen ist und die Markierung (24, 32, 32b, 42, 42b) aus röntgendichtem Material hergestellt ist.

11. Ummantelter Stent nach Anspruch 10, wobei der ummantelte Stent ein zusätzliches Merkmal e), f) oder g) aufweist:
e) die Markierung ist in Umfangsrichtung an der inneren Öffnung oder der äußeren Öffnung angeordnet;
f) der äußere Zweig oder der mindestens eine innere Zweig (3b, 4b) ist mit der Markierung (32b, 42b) entlang einer Erstreckungsrichtung davon versehen;
g) die Markierung ist auf den äußeren Zweig (2) und den inneren Zweig (3, 32, 4, 42) heißgepresst oder genäht.

12. Ummantelter Stent nach Anspruch 1, wobei der ummantelte Stent ein zusätzliches Merkmal n) aufweist:
n) der proximale Röhrenkörper (11) ist mit einem letzten Freigabeabschnitt (105) versehen, der eine radial zusammenfaltbare Ringstruktur ist, die an der Abdeckmembran (102) befestigt ist und sich proximal über eine proximale Kante der Abdeckmembran (102) hinaus erstreckt.

13. Ummantelter Stent nach Anspruch 12, wobei der letzte Freigabeabschnitt (105) mit vorstehenden Widerhaken versehen ist, die in Umfangsrichtung angeordnet sind.

14. Ummantelter Stent nach einem der Ansprüche 1 bis 13, wobei eine röntgendichte Markierung (106) an einem Ende der Abdeckmembran (102) vorgesehen ist und die röntgendichte Markierung (106) aus röntgendichtem Material hergestellt ist.

## Revendications

1. Endoprothèse couverte, comprenant :
un corps d'endoprothèse (1, 1a, 1c, 1d, 1e, 1f) ayant une structure tubulaire avec une membrane de couverture (102) sur une surface de celui-ci, et formant une partie concave radialement évidée (103) sur celui-ci ;
une branche externe (2, 2a, 2c, 2d, 2e, 2f) s'étendant à l'extérieur du corps d'endoprothèse (1, 1a, 1c, 1d, 1e, 1f), ayant une extrémité raccordée à une paroi latérale de la partie concave (103) avec une ouverture interne (21, 21a, 21f) sur la paroi latérale de la partie concave (103) ; et
au moins une branche interne (3, 3a, 3b, 3c, 3d, 3e, 3f, 4, 4a, 4b, 4c, 4d, 4e, 4f), fixée à une paroi intérieure du corps d'endoprothèse (1, 1a, 1c, 1d, 1e, 1f), chacune de l'au moins une branche interne (3, 3a, 3b, 3c, 3d, 3e, 3f, 4, 4a, 4b, 4c, 4d, 4e, 4f) ayant une extrémité raccordée à la paroi latérale de la partie concave (103) et avec une ouverture externe (31, 31a, 31c, 31d, 41, 41c, 41d) sur la paroi latérale de la partie concave (103),
**caractérisée en ce que** le corps d'endoprothèse (1, 1a, 1c, 1d, 1e, 1f) comprend un corps de tube proximal (11), un corps de tube médian (12), et un corps de tube distal (13) en séquence d'une extrémité proximale à l'extrémité distale ; chacun du corps de tube proximal (11), du corps de tube médian (12) et du corps de tube distal (13) est une structure tubulaire avec un diamètre sensiblement constant, un diamètre du corps de tube médian (12) est inférieur à un diamètre du corps de tube proximal (11) et à un diamètre du corps de tube distal (13), et le corps de tube médian (12) constitue la partie concave (103),
dans laquelle deux extrémités du corps de tube médian (12) sont respectivement raccordées au corps de tube proximal (11) et au corps de tube distal (13) par deux corps de tube de transition (14, 15) ; chacun des corps de tube de transition (14, 15) a un cadre de support de transition effilé (104) pour supporter la membrane de couverture (102), et une extrémité plus petite du cadre de support de transition (104) fait face au corps de tube médian (12),
dans laquelle l'une extrémité de la branche externe (2, 2a, 2c, 2d, 2e, 2f) est raccordée à une paroi latérale du corps de tube médian (12), avec l'ouverture interne (21, 21a, 21f) définie sur la paroi latérale du corps de tube médian (12), et l'une extrémité de chaque branche interne (3, 3a, 3b, 3c, 3d, 3e, 3f, 4, 4a, 4b, 4c, 4d, 4e, 4f) est raccordée à la paroi latérale du corps de tube médian (12), avec l'ouverture externe (31, 31a, 31c, 31d, 41, 41c, 41d) définie sur la paroi latérale du corps de tube médian (12),
dans laquelle l'ouverture externe (31, 31a, 31c, 31d, 41, 41c, 41d) est plus proche d'une extrémité distale que l'ouverture interne (21, 21a, 21f).

2. Endoprothèse couverte selon la revendication 1, dans laquelle au moins une branche interne (3, 4) comprend deux branches internes (3, 3a, 3b, 3c, 3d, 3e, 3f, 4, 4a, 4b, 4c, 4d, 4e, 4f), qui sont une première branche interne (3, 3a, 3b, 3c, 3d, 3e, 3f) et une seconde branche interne (4, 4a, 4b, 4c, 4d, 4e, 4f) respectivement, dans laquelle la première branche interne (3, 3a, 3b, 3c, 3d, 3e, 3f) a une première ouverture externe (31, 31a, 31c, 31d) sur la paroi latérale de la partie concave (103), la seconde branche interne (4, 4a, 4b, 4c, 4d, 4e, 4f) a une seconde ouverture externe (41, 41c, 41d) sur la paroi latérale de la partie concave (103), et la seconde ouverture externe (41, 41c, 41d) est plus proche de l'extrémité distale que la première ouverture externe (31, 31a, 31c, 31d).

3. Endoprothèse couverte selon la revendication 2, dans laquelle la première branche interne (3, 3a, 3b, 3c, 3d, 3e, 3f) s'étend depuis la première ouverture externe (31, 31a, 31c, 31d) vers une extrémité proximale.

4. Endoprothèse couverte selon la revendication 3, dans laquelle l'endoprothèse couverte ((1, 1a, 1c, 1d, 1e, 1f)) a une caractéristique supplémentaire a) ou b) :
a) la seconde branche interne (4, 4a, 4b, 4c, 4e, 4f) s'étend depuis la seconde ouverture externe (41, 41c) vers l'extrémité distale ;
b) avec une extension vers l'extrémité proximale, la première branche interne (3a, 3b, 3c, 3d) s'étend en outre dans la direction circonférentielle du corps d'endoprothèse depuis la première ouverture externe (31a, 31c, 31d).

5. Endoprothèse couverte selon la revendication 4 avec la caractéristique a), dans laquelle avec une extension vers l'extrémité distale, la seconde branche interne (4c) s'étend en outre dans une direction circonférentielle du corps d'endoprothèse depuis la seconde ouverture externe (41c).

6. Endoprothèse couverte selon la revendication 4 avec la caractéristique b), dans laquelle la seconde branche interne (4d) s'étend depuis la seconde ouverture externe (41d) vers l'extrémité proximale, et avec une extension vers l'extrémité proximale, la seconde branche interne (4d) s'étend en outre dans la direction circonférentielle du corps d'endoprothèse (1d) depuis la seconde ouverture externe (41d) ; et une direction d'extension circonférentielle de la seconde branche interne (4d) est opposée à une direction d'extension circonférentielle de la première branche interne (3d).

7. Endoprothèse couverte selon l'une quelconque des revendications 1 à 6, dans laquelle une longueur de la branche externe (2, 2a, 2c, 2d, 2e, 2f) est de 5 mm à 20 mm.

8. Endoprothèse couverte selon l'une quelconque des revendications 1 à 7, dans laquelle la branche externe (2f) a une section flexible (221f) capable d'une déformation flexible, et la section flexible (221f) s'étend vers l'extérieur depuis l'ouverture interne (21f).

9. Endoprothèse couverte selon la revendication 8, dans laquelle l'endoprothèse couverte a une caractéristique supplémentaire c) ou d) :
c) la section flexible (221f) est constituée d'un matériau de membrane flexible ;
d) une longueur de la section flexible (221f) est de 2 mm à 10 mm, sans dépasser 1/2 de la longueur totale de la branche externe (2f).

10. Endoprothèse couverte selon l'une quelconque des revendications 1 à 9, dans laquelle chacune de la branche externe (2) et de la branche interne (3, 3b, 4, 4b) est munie d'un marqueur (24, 32, 32b, 42, 42b), et le marqueur (24, 32, 32b, 42, 42b) est constitué d'un matériau radio-opaque.

11. Endoprothèse couverte selon la revendication 10, dans laquelle l'endoprothèse couverte a une caractéristique supplémentaire e), f) ou g) :
e) le marqueur est agencé circonférentiellement au niveau de l'ouverture interne ou de l'ouverture externe ;
f) la branche externe ou l'au moins une branche interne (3b, 4b) est munie du marqueur (32b, 42b) le long d'une direction d'extension de celle-ci ;
g) le marqueur est pressé à chaud ou suturé sur la branche externe (2) et la branche interne (3, 32, 4, 42).

12. Endoprothèse couverte selon la revendication 1, dans laquelle l'endoprothèse couverte a une caractéristique supplémentaire n) :
n) le corps de tube proximal (11) est muni d'une partie de libération de dernier (105) qui est une structure annulaire radialement pliable fixée à la membrane de couverture (102), et s'étendant de manière proximale au-delà d'un bord proximal de la membrane de couverture (102).

13. Endoprothèse couverte selon la revendication 12, dans laquelle la partie de libération de dernier (105) est munie de barbes saillantes agencées circonférentiellement.

14. Endoprothèse couverte selon l'une quelconque des revendications 1 à 13, dans laquelle un marqueur radio-opaque (106) est prévu au niveau d'une extrémité de la membrane de couverture (102), et le marqueur radio-opaque (106) est constitué d'un matériau radio-opaque.
